# EUROPEAN PATENT SPECIFICATION

(11) **EP 1 547 572 B1**
(45) Date of publication and mention of the grant of the patent: **15.08.2007**
(21) Application number: 03029538.0
(22) Date of filing: 22.12.2003
(51) Int. Cl.: A61K 6/027, C03C 13/00, C03C 13/04, C03C 21/00, A61K 6/083

(54) **Glass filler material and method of production**
Glasfüllmaterial und Verfahren zur Herstellung
Matériau de verre d'obturation et procédé de production

(43) Date of publication of application: 29.06.2005
(73) Proprietor: 3M ESPE AG, 82229 Seefeld (DE)
(72) Inventor: Hoescheler, Stefan, D-82211 Herrsching (DE); Albrecht, Dirk, D-82211 Herrsching (DE); Stippschild, Andrea, D-86899 Landsberg (DE); Dede, Karsten, D-86899 Landsberg (DE)
(74) Representative: Vossius & Partner

(56) References cited:
- EP-A- 0 102 199
- WO-A-96/29283
- US-A- 4 376 835
- US-A- 4 900 697
- US-A- 5 147 904

## Description

The present invention generally relates to a glass filler material. More specifically the present invention discloses a glass filler material for composites with cationically curing properties and for dental composite materials.

The glass filler material does not influence the curing properties of the composite and does not deteriorate the mechanical properties of the cured plastic polymer (i.e. a dental filling) and does not decrease the shelf life stability of the composite.

This can be reached by a glass filler material wherein the particles of this material have an inner zone and an outer zone wherein the outer zone is almost free of alkali metal oxides and the alkali metal oxides of the inner zone do not migrate to the outer zone.

The present invention further relates to a method for producing a glass filler material which does not influence the curing properties of the composite and does not deteriorate the mechanical properties of the cured plastic polymer and does not decrease the shelf life stability of the composite. More specifically it relates to a method for producing a glass filler material for composites with cationically curing properties and more specifically for dental composites.

The term "polymerizable resin" means the monomer or the mixture of monomers which undergo polymerization by adjacent initialization. The polymerizable resin may contain a certain amount of prepolymerized oligomers and/or polymers based on the monomers.

The polymerizable resin often is mixed with a filler material to enhance the properties of the uncured material. In the following this material is called "composite" or "composite material", i.e. dental composite material.

By terms of this invention "plastic polymer" means the cured material after polymerization containing no or only small amounts of monomers. The resulting plastic polymer obtained after appropriate curing even exhibits enhanced properties by adding a filler to the polymerizable resin.

Glass filler materials for curable composites are disclosed in numerous documents.

EP 716 049 A2 discloses a barium-free dental glass with good X-ray absorption properties comprising the following (in wt.%): 50-75 silica, 5-30 zirconia, 0-5 lithium oxide, 0-25 sodium oxide, 0-25 potassium oxide and 0-25 alkali metal oxides (weight relative to oxides).

EP 634 373 discloses a barium-free dental glass with good X-ray absorption properties comprising the following (in wt.%): 45-65 silica, 5-20 boron oxide, 5-20 aluminum oxide, 0-10 calcium oxide, 15-35 strontium oxide, 0-2 fluorine.

US 6,270,562 B1 relates to a filler material for use in dental composites and dental restorations comprising a fibrous material and one or more forms of surface-modifying particles. The surface-modifying particles are bonded to the fibrous material to increase the surface area of the fibrous material and improve the bonding properties of the fibrous material to enable it to better bond to a resin matrix material in a dental composite.

WO 99/20225 describes a method of making a composition for forming a dental composite material comprising a glass fiber filler. These fibers are obtained by grinding glass fibers which have been densified and embrittled by heating glass fibers at a temperature substantially below the softening point of the glass fibers.

US 6,022,819 relates to a porcelain composition comprising in weight percent 50 - 85 % SiO₂, 2 - 18 % Al₂O₃ and 2 - 23 % of a flux. The flux is selected from the group consisting of K₂O, Na₂O, Li₂O, CaO, P₂O₅, F, BaO, B₂O₃ and mixtures thereof.

EP 997 132 A1 discloses a X-ray opaque barium-free dental glass comprising the following (in wt.%): 20 - 45 silicon dioxide, 5 - 35 aluminum oxide, 2 - 20 zinc oxide, 2 - 10 zirconium oxide, 2 - 10 fluorine and 1 - 10 sodium oxide.

DE 198 46 556 describes dental materials based on polymerizable monomers, epoxides, organic modified polysiloxanes, liquid crystal monomers, oxethanes, spiro-ortho esters or carbonates as binders, a catalyst for hot, cold or photopolymerization, 20-70 wt. % inorganic filler (A), 0-60 wt. % other fillers (B) and 0-2 wt. % conventional additives. The inorganic filler (A) consists of a porous glass ceramic having micro- and/or meso-pores filled with the binders optionally in polymerized form.

WO02/05502 A2 describes a polymerizable dental material with a filler material. The filler material is produced by means of a melting method and is selected so that it has a refractive index of n_{D} = 1.49 to 1.54 so that the viscosity of the polymerizable dental material, after a period of at least 9 month during which it was stored at a temperature ranging form 20 to 25 °C, has a value of +/- 50 % of the initial value measured 24 hours after the polymerizable dental material was produced, and so that the polymerizable dental material has a reactivity of the type that, once polymerization is initiated, the amount of the maximum heat flux generated by the dental material equals at least 0.8 mW/mg, and this maximum heat flux is attained within a period of no longer than 60 seconds.

EP 023 013 B1 relates to a calcium aluminium fluorosilicate glass powder having an average particle size of at least 0,5 µm wherein the powder particles are so depleted of calcium at their surface that the quotient of the atomic ratio Si/Ca at the surface of the powder particles and the atomic ratio Si/Ca in the core region is at least 2.0. It further describes a method to remove these ions from the particle surface up to a depth of about 50 nm. This calcium aluminium fluorosilicate glass powder is used for a glass ionomer cement.

A method for preparing glass substrates is disclosed in EP 819 103. In one step the surface of a silica-soda-lime glass substrate is treated to a first depth with an ion-exchange treatment for a sufficient first time and first temperature to provide an ion-exchanged treated glass having a strengthened surface. In a following step the surface of the ion-exchanged treated glass is treated to a second depth with a dealkalization treatment for a sufficient second time and second temperature to remove alkaline ions from the glass surface, wherein the second depth is less than the first depth. In this disclosure the method of dealkalization is only useful to remove the ions to a depth of less than 1 µm. It further needs temperatures of more than 100 °C. The dealkalization is carried out with AlCl₃ or (NH₄)₂SO₄. Fillers treated with these reagents are not usable for the composites described herein as they result in other problems of the filled composites like short storage time. The dealkalization is described only for hard surfaces like disks and not for powder particles.

A further method of making dealkalized glass is disclosed in DE 37 41 031 A1. The glass is brought into contact with the acidic gas of a dealkalizing medium. It is especially useful for products with coated glass parts as mirrors.

Glass filler materials are used to increase the mechanical properties of plastic polymers. Through the addition of glass filler materials to polymerizable resins the resulting composite materials exhibit good handling characteristics as they are formable and do not stick to the handling tools. The cured plastic polymers of these filled composite materials further show improved strength, elastic modulus, hardness and wear resistance for the cured plastic polymer. Such composites are used in electronical engineering, precision work technique, for constructing of though housings, in household and in medical applications, for example as joint implants or as dental materials.

For dental composite materials the fillers have to meet further requirements as X-ray opacity, dielectrical properties, biocompatibility and a certain refractive index. In order to obtain dental materials with a desirable translucency in visible light this refractive index should be close to the refractive index of the polymerizable resin.

The polymerizable resin composition further demands certain chemical properties of the filler. This means that the filler should not influence the polymerization reaction and should not interact with the initiator system. The polymerization is started in different ways for example by mixing a base part of the resin with an initiator containing part of the resin (two component system) or by exposing light to the resin containing a light sensitive initiator system. The initiator systems used in the state of the art react on a radical basis or on an ionic, preferably a cationic basis. Thus, they are sensitive to the presence of water, basic substances or acids.

Mainly used filler materials are quartz or glass. Because of the defined chemical composition, the quartz material has a refractive index of n_{D} = 1.55. For a lot of polymerizable resins with different refractive indices, a filler with this index is only minor suitable as a translucent composite is not available. For a glass filler, the reasonable refractive index of the filler is adjustable by the adjacent composition of the glass.

To obtain a glass filler material, the main components as SiO₂, B₂O₃, P₂O₅ are mixed together with other oxides, hydroxides or carbonates of elements of group I and II , of transition elements or of lanthanides. The mixture is melted to the glass at temperatures of 1250 to 1650 °C. Of importance are the components with elements of the main group I (Li, Na, K, Rb, Cs) which significantly decrease the melting temperature as well as the viscosity of the melted glass. Thus, for the glass filler materials known from the state of the art, the amount of these elements normally ranges between 5 and 40 mol%.

It is further known that different acidic ingredients as B₂O₃ or P₂O₅ also decrease the melting temperature of the glass mixture.

The presence of acidic, amphoteric or basic oxides is a disadvantage for the fillers as these glass filler materials do not have the demanded chemical properties. When used as a filler of polymerizable resins the acidic, amphoteric or basic oxides do interact with the monomers or with the initiators systems. The use of glass filler materials with the elements of the main group I in the said concentrations in composites results in low mechanical properties of the cured plastic polymer. An amount of acidic or amphoteric oxides as disclosed in the state of the art leads to a shortened storage time of the composites. The storage time is especially decreased for composites with cationically curing monomers.

If a glass material is desired without these elements of main group I or the acidic ingredients the melting process has to be carried out at very high temperatures. This high temperature melting method is time consuming and very expensive. The obtained glass materials have the tendency to crystallize what is not acceptable for fillers used in dental materials.

It is thus an object of the present invention to avoid one or more of the problems mentioned above.

A further object is to provide a new glass filler material for composite materials and to provide a glass filler material with improved properties. Another further object is to provide a glass filler material with a concentration of alkali metal oxides which allows to melt the glass filler material at suitable temperatures and which allows at the same time a composite material with good polymerization properties and good mechanical properties of the cured plastic polymer.

A further object is to provide a method of producing such a glass filler material. Still a further object is to provide a dental composition containing these glass filler materials.

One or more objects can be achieved by providing a glass filler material as described in the text below.

According to the present invention the glass filler material comprises
a) 65 - 99.95 mol% silicon dioxide (SiO₂),
b) 0 - 15 mol% aluminum and/or boron oxide (Al₂O₃ B₂O₃),
c) 0 - 30-mol % zirconium and/or titanium and/or hafnium oxide (ZrO₂, TiO₂, HfO₂), Y₂O₃ and/or Sc₂O₃ and/or La₂O₃ and/or CeO₂ and/or other lanthanideoxides
d) 0.05 - 4 mol% alkali metal oxides (Na₂O, Li₂O, K₂O, Rb₂O, Cs₂O)
e) 0 - 25 mol% earth alkali metal oxides (MgO, CaO, SrO, BaO).

The glass filler particles have an average particle size of 0.1 - 20 µm and preferable the average particle size is from 0.5 to 3 µm and more preferable from 0.5 to 1 µm. Within these preferred particles sizes the particles of the invention have an inner zone and an outer zone where the outer zone is up to 1.5 µm. In some cases the thickness of the outer zone could even be about 2 µm or more.

It is the intention of this invention to differentiate the inner zone of the particles from the outer zone by the profile of alkali ion concentration or even alkali oxide concentration versus the layer depth of the particle.

This profile is attachable by an analysis of the alkali concentration of the surface of the particles by a suitable method. The method used for this invention is the photoelectron spectroscopy (ESCA). This method of analysis describe R.S. Swingle II and W.M. Riggs in "Critical review in Analytical Chemistry" (Volume 5, Issue 3, pages 267 - 321, 1975) as well as K. Levsen in "Chemie unserer Zeit" ( 10th annual, 1976, no. 2, pages 48-53).

The particle profile shows the concentration [mol%] of Na+ or other alkali ions on the y-axis versus the layer depth of the particle in [nm] on the x-axis starting at 0 nm indicating the surface of the particle. Normally the profile is detected to a depth corresponding to the radius of the particle or less than this radius. It usually has a significant increase of the alkali ion concentration when passing from the outer zone to the inner zone of the particle. The layer depth in the maximum of the first derivation of the concentration to the layer depth ( f' = d(conc)/d(depth) ) represents the thickness of the outer zone. The maximum is characterized in that the second derivation becomes 0 (f" = 0) at the same value of depth.

A common profile of a particle with an outer zone of about 850 nm and a diameter of 4 µm or more is shown in the figure 1:

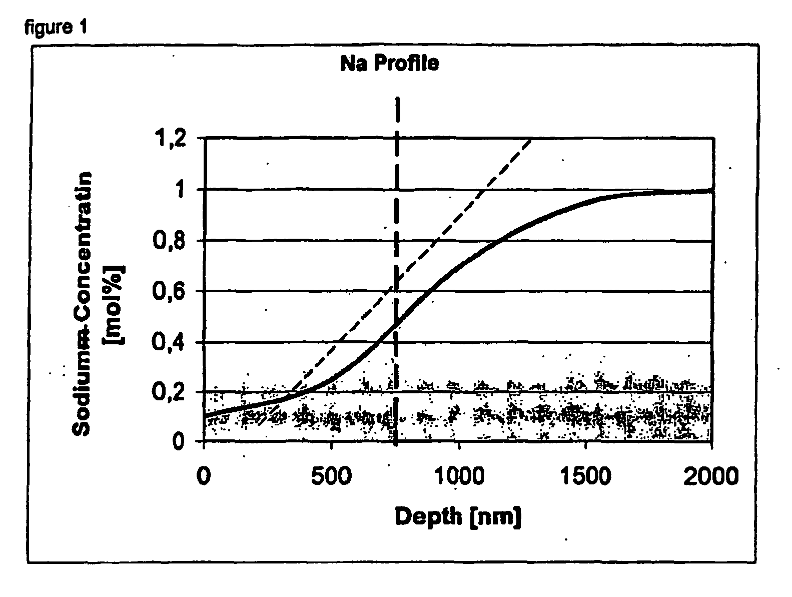

The depth of the outer zone of the particles is preferably about 1.5 µm even for very large particles. It could be larger depending on which alkali ion is present in the glass filler material.

For these particles with diameters smaller than 3 µm nearly the whole particles consist of the outer zone and possibly no inner zone is detectable by the ESCA method.

For the particles according to the invention the mean concentration of alkali ions of the outer zone relative to the mean concentration of alkali ions of the inner zone is 10 % or less and the alkali ions of the inner zone do not significantly migrate to the outer zone. It doesn't make any difference for the invention if the concentration of alkali metal oxides of the particles is counted in oxides or in cations. Despite it is very clear that for the migration process in filler materials only the cations are of interest as only the ionic parts of the oxides are subjected to any detectable migration.

The limitation that the alkali ions do not migrate "significantly" is based on the fact that it is not possible to totally exclude any motions of the ions especially if these motions are very limited and small. But it is an important feature of the invention that almost no migration takes place. This means for example that only 0.5% of the alkali ions of the inner zone migrate after a time of storing the filler material for nine month at a temperature of 25 °C. It is most preferred that only 0.1 % of the alkali ions migrate to the outer zone under these storage conditions.

To provide glass filler materials which result in composites with good mechanical properties and an adequate storing it is preferred that the filler material has an amount of alkali metal oxides from 0.05 to 2 mol%. Most preferably the concentration of alkali metal oxides in the filler material is not over 1 mol%. The claimed amount of alkali metal oxides is the total amount of the particle without respect to the different concentrations of the inner and the outer zone of the particles.

The maximal particle size of the glass filler material is 100 µm. More preferred is a maximal particle size of 5µm. In case the glass filler material is used as a filler of a dental cement the maximal particle size is 25µm, preferably 20 µm. To reach excellent mechanical properties of the cured plastic polymer the statistic distribution of the particle size is not too narrow. This appropriate distribution is available by the known milling processes and the separation of the coarse grained fraction.

The filler material of the invention has a refractive index (n_{D}) of 1.49 to 1.55. The refractive index is elected in that way that it is close to the refractive index of the polymerizable resin.

The glass filler material of the invention is used in the dental restorative field in composite formulations, especially for fillings, bondings, dental cements, pit and fissure sealeants, cavity lining, core build up.

Further the fillers could be use for materials for temporary crowns and bridges, for root fillings, for sub fillings, for dental protheses materials such as inlays, onlays, crowns, bridges and for a denture material.

Most favorable is their uses in polymerizable materials and especially in composites with cationically curing properties.

They may be even useful for other dental materials as glass ionomer cements, compomers, blending material for crowns or bridges, ceramics.

The described glass filler material is available by the following method.
a) melting a composition of 65 - 99.95 mol% SiO₂, 0 - 15 mol% Al₂O₃ and/or B₂O₃, 0 - 30 mol% ZrO₂ and/or TiO₂ and/or HfO₂, 0.05 - 4 mol% alkali metal oxides, 0 - 25 mol% earth alkali oxides, 0 - 30 mol% Y₂O₃ and/or Sc₂O₃ and/or La₂O₃ and/or Ce₂O₃ and/or other lanthanidoxides at a temperature of 1200 - 1800 °C for at least 30 minutes,
b) crushing the melted glass by transferring into cold water or on metal rollers,
c) milling the glass granulate obtained by b) to a mean particle size d₅₀ from 0.1 to 20 µm,
d) dealkalizing the glass powder in excess with a dealkalizing agent,
e) removing the dealkalizing agent and washing the glass powder with a polar solvent until the filtrate reacts neutral,
f) drying the glass powder at a temperature of 200 - 1100 °C for at least 30 minutes.

The best melting temperature depends on the amount of the different ingredients. Especially Al₂O₃, B₂O₃ and alkali metal oxides help to decrease the melting temperature of the mixture. Preferable the melting temperature is from 1400 to 1700 °C and more preferably from 1450 to 1550 °C.

The melted glass is crushed. This could be done by transferring it into cold water or on metal rollers. At the same time, the glass melt is cooled. The whole melting and crushing procedure could be carried out in a discontinuous or a continuous process. A possible melting crucible is a platinum crucible.

After crushing the melt the obtained glass granulate is milled to a mean particle size of d₅₀ from 0.1 to 20 µm. For the pre-milling of the crushed glass to a particle size of about 300 µm an agate disc mill could be used. Preferred milling methods for fine milling particles with an average size of 300 µm and smaller into the range of d₅₀ < 20 µm are ball mills. In these mills the preferred balls are yttrium-stabilized ZrO₂-ball with a diameter of 0.8 mm. The milling container of these ball mills could be coated with Al₂O₃ .

The dealkalizing agent should be an acidic composition able to solve alkaline ions. Examples for a dealkalizing agent are inorganic or organic acids as HCl, HJ, HBr, H₂SO₄, H₃PO₄, HNO₃, HClO₄, CH₃COOH, COOH-COOH, H-COOH, citric acid, tartaric acid or polycarboxylic acid. These acids are used in concentrations of 10 to 30% acid in water. Preferred examples are 10% CH₃COOH, 10 % HCOOH, 30% HCI or 15% HNO₃ . Mixtures of the described acids could be used as well. The dealkalizing step is performed at a temperature of 50 to 200 °C. Most preferred is a temperature of 100 to 120 °C. The dealkalizing agent is used in excess. This surplus ratio of dealkalizing agent to the glass powder to be dealkalized is important for the invention.

It is a feature of this invention that the ratio of the glass powder to the dealkalizing agent is 1:1 to 1:1000. Preferably the ratio is 1:10 and more preferably 1:20.

Surprisingly with this dealkalizing step a glass filler material is obtained with an outer zone of up to 1.5 µm wherein this outer zone is almost free of alkali ions. It is further not expected that the alkali of the inner zone do not significantly migrate to the outer zone. The principles of the balance between different concentrations will assume that a migration occurs from the zone with the higher concentration to the zone with the lower concentration. Despite this, no significant migration between the two zones occurs.

The polar solvent for washing the dealkalized glass powder consists of water or a mixture of water with other polar solvents, preferably ethanol or acetone. The washing step could take place at different temperatures depending on the polar solvent. It is preferably performed at room temperature.

The drying of the glass powder could be done at a temperature of 200 to 1100 °C for at least 0.5 hours. It must be a temperature clearly below the sintering temperature of the glass composition in order to avoid the sintering of the particles. This temperature varies for the different compositions of glasses. A temperature of 500 to 1000 °C is useful of most of the glass compositions and a temperature of 800 to 1000 °C is preferred.

After drying a sieving process could be added to remove the coarse fraction of the particles. For example a 200 µm meshed screen. This sieving step is not mandatory.

The particles obtained by the described method have an average particle size d₅₀ from 0.1 to 20 µm. Preferably they have an average particle size d₅₀ from 0.5 to 3 µm and more preferably from 0.5 to 1 µm.

Of these glass filler materials a polymerizable dental material is available containing:
a) 3 - 80 wt.% of one or more cationically curable monomers,
b) 3 - 90 wt.% of the glass filler material of the invention,
c) 0 - 90 wt.% of one or more radio-opaque fillers,
d) 0.01- 25 wt.% of initiators, retarders and/or accelerators,
e) 0 - 25 wt.% of auxiliary agents

The curable monomers are selected for example from epoxy, oxetane, vinyl ether and spiro-orthocarbonate resins, and combinations thereof. Preferably, the cationically polymerizable monomers comprise an epoxy resin, especially a silicon-containing epoxy resin, or a blend of a silicon-containing epoxy resin and an epoxy resin that does not contain silicon.

Suitable fillers for radiopaque formulations are described in EP-A2-0 189 540, EP-B-0 238 025, and U.S. Patent No. 6,306,926 B1.

As initiators are possible for example systems comprising an iodonium salt and a visible light sensitizer, and optionally an accelerator. The iodonium salt may be a diaryl iodonium salt such as diaryliodonium hexafluorophosphate, diaryliodonium hexafluoroantimonate, 4-octyloxyphenyl phenyliodonium hexafluoroantimonate, 4-(2-hydroxytetradylecoxyphenyl) phenyliodonium hexafluoroantimonate, 4-(1-methylethyl)phenyl 4-methylphenyliodonium tetrakis(pentafluorophenyl)borate, and combinations thereof.

The visible light sensitizer may be selected from ketones, coumarin dyes, xanthene dyes, fluorone dyes, fluorescein dyesaminoketone dyes, p-substituted aminostyryl ketone compounds, and combinations thereof. More preferably, the visible light sensitizer is an alpha-diketone; camphorquinone is particularly preferred.

The accelerators may be selected from the group of polycyclic aromatic compounds.

The polymerizable dental materials which could contain the glass filler material of the invention are for example described in WO 98/47046, WO 01/51540 A2, WO 98/22521 and WO 02/055028 A2 which are incorporated to this disclosure by reference. Most preferred are the polymerizable materials containing cationically curable monomers as composite.

The glass filler material of the invention as obtained by the described method is used as mentioned above in the dental field. In the dental restorative field it is used in favor for composite formulations, especially for fillings, bondings, dental cements, pit and fissure sealeants, cavity lining, core build up. Most favorable is their uses in polymerizable materials and especially in composites with cationically curing properties.

### Examples:

For all examples conventional raw materials for glass melting as oxides, carbonates or hydroxides of the corresponding elements are used. Shown in the following table 1 are the compositions of the melted glass mixtures calculated as oxides of these elements as they result after melting. (all oxides in mol%, up to an amount of 150.0 g for each example):

**table 1**

| **Example no.:** | **1** | **2** | **3** | **4** | **5** | **6** | **7** | **8** | **9** | **10** |
|---|---|---|---|---|---|---|---|---|---|---|
| SiO₂ | 70 | 75 | 70 | 74 | 80 | 75 | 85 | 85 | 73 | 73 |
| Li₂O | | | 20 | | | 14 | | | 5 | 14 |
| Na₂O | 16 | 11 | | 16 | 12 | | 12 | 15 | 8 | |
| K₂O | | | | | 4 | | | | | |
| Al₂O₃ | 2 | | | | | | | | | 1 |
| B₂O₃ | | 4 | | | | | | | | |
| MgO | 2 | | | | | | | | 2 | |
| SrO | | | | | | | | | 2 | |
| La₂O₃ | | | | | | | | | 10 | |
| Y₂O₃ | | 10 | | | | 11 | | | | 6 |
| ZrO₂ | 10 | | 5 | 10 | 4 | | 3 | | | 6 |
| TiO₂ | | | 5 | | | | | | | |

For each example 1 to 10 the raw materials like oxides, carbonates and/or hydroxides in an amount and a ratio to give 150 g of the above mentioned molar compositions in the resulting glass melt are mixed together. The mixture for each example is melted in a 400 ml platinum crucible (PT10Rh) at a temperature of 1500 to 1640 °C. For this application in a laboratory dimension a discontinuous process is used. For a larger amount of materials a continuous process is more useful. After a melting time of 1 to 2 hours the melted glass is quenched by bringing it into a stainless steel vessel with 10 I distilled water.

The glass material of each example is milled in a first step in an agate disc mill to a powder with a particle size of d₅₀ < 300 µm. In a second step the premilled powder is fine milled in a ball mill. Therefore, 150 g of the premilled powder, 200 ml isopropylic alcohol and 1100 g of yttrium-stabilized balls made of ZrO₂ are brought into a vessel of a volume of 1 liter. The balls have a diameter of 0.8 mm. The vessel is coated with Al₂O₃ . The milling step is performed until the powders have an average particle size d₅₀ of 0.6 to 1 µm.

After milling, the powders of example 1 to 3 are treated with 15% HNO₃, the powder of example 4 is treated with 10 % CH₃COOH, the powders of example 5 to 8 are treated with 10% CHOOH and the powders of example 9 and 10 are treated with 30% HCl. All examples are dealkalized with the mentioned acids at a temperature of 100 to 120 °C under heating and stirring in a reflux condenser for 16 hours. The ratio of powder to fluid for all examples is 1:15.

After dealkalizing each powder the dealkalizing agent is removed. The acid-powder-mixture is filtered in a pressure filter funnel with a fritted disc containing a 0.4 µm PTFE(Teflon®) membrane. For washing the amount of 150 g of each powder an amount of 10 I distilled water is used.

Then each powder is brought into a Al₂O₃ crucible and dried at a temperature of 600 °C in a circulation oven, except the powder of example 4 which is dried at 920 °C.

The dried powders are sieved through a 200 µm meshed screen.

The following table 2 shows the composition of the glass filler materials in mol% of example 1 to 10 after the described process. The analysis is performed by ICP (Fraunhofer Gesellschaft Germany) :

**table 2**

| **example no.**: | **1** | **2** | **3** | **4** | **5** | **6** | **7** | **8** | **9** | **10** |
|---|---|---|---|---|---|---|---|---|---|---|
| SiO₂ | 85.0 | 86.1 | 86.2 | 85.9 | 94.4 | 86.2 | 96.1 | 99.2 | 88.1 | 84.9 |
| Li₂O | | | 1.2 | | | 1.1 | | | 0.3 | 1.0 |
| Na₂O | 0.8 | 0.7 | | 1.5 | 0.6 | | 0.5 | 0.8 | 0.4 | |
| K₂O | | | | | 0.2 | | | | | |
| Al₂O₃ | 1.2 | | | | | | | | | 1.0 |
| B₂O₃ | | 2.0 | | | | | | | | |
| MgO | 1.2 | | | | | | | | 1.0 | |
| SrO | | | | | | | | | 1.2 | |
| La₂O₃ | | | | | | | | | 9.0 | |
| Y₂O₃ | | 11.2 | | | | 12.7 | | | | 6.1 |
| ZrO₂ | 11.8 | | 6.2 | 12.4 | 4.8 | | 3.4 | | | 7.0 |
| TiO₂ | | | 6.4 | | | | | | | |

In table 3 the physical properties of the glass filler materials are listed. The refractive index is defined by the immersion method (Infracor Hanau). The pH value of the glass filler materials is measured before and after the dealkalizing step to show the magnitude of migration of the basic oxides out of the outer zone. Therefore, 1 g of the powder is dispersed in 100 ml distilled water with a magnet stirrer. The pH-value is taken with an H⁺-electrode after a constant value appeared. To measure the X-ray opacity a composite material containing the glass filler material is prepared. Therefore,

| | |
|---|---|
| 30 g | 1,3,5,7-Tetrakis(2,1 -ethanediyl-3,4-epoxycyclohexyl)-1,3,5,7-tetramethyl-cyclotetrasiloxane, |
| 1.8 g | Tolylcumyliodonium-tetrakis(pentafluorophenyl)borate, |
| 0.2 g | 2-Butoxyethyl-4-(dimethylamino)benzoate, |
| 0.5 g | Camphorquinone |

are mixed together under light exclusion. Then, 72 g of the glass filler material according to example 1 to 10 are kneaded into the monomer composition. The resulting composite is brought into round test platelets (diameter 1.5 cm and thickness 1.5 mm) of the material cured by light exposure with Elipar Freelight ® for 40 s. The resulting cured plastic polymer platelets are measured according to ISO 4049.

**table 3**

| **example no.:** | **1** | **2** | **3** | **4** | **5** | **6** | **7** | **8** | **9** | **10** |
|---|---|---|---|---|---|---|---|---|---|---|
| refractive index n_{D} | 1.53 | 1.52 | 1.53 | 1.54 | 1.50 | 1.52 | 1.49 | 1.47 | 1.52 | 1.52 |
| pH before dealkalizing | 11 | 11.5 | 12 | 10.5 | 11 | 11 | 11.5 | 11 | 11 | 11.5 |
| pH after dealkalizing | 8 | 8.5 | 8.5 | 7.5 | 7.5 | 7 | 7.5 | 7.0 | 7.5 | 8 |
| X-ray opacity | 200 | 200 | 150 | 120 | 190 | 100 | 80 | 190 | 200 | 140 |

All glass filler materials of examples 1 to 10 show a refractive index between 1.47 and 1.54 which is in the range to get a translucent cured plastic polymer with most of the common polymerizable resins. All glass fillers exhibit a sufficient X-ray opacity after incorporating them into a composite material.

The mean molar concentration of alkali ions (Li⁺, Na⁺ and K⁺) in the outer zone of the particles (surface layers up to a depth of about 2 µm) before and after storage (9 month, 25°C) was investigated by ESCA. The same investigation was performed for the inner zone of the particles (deeper layers in a depth of 3 µm and more).

With ESCA, a concentration profile showing the alkali ion concentration in a certain depth of the particle (in nm) was monitored. Out of this profile the boundary between inner and outer zone were easily defined. For each zone the average molar concentration of alkali ions was calculated by integration over the profile.

The mean concentrations of alkali ions (Na⁺ + Li⁺ + K⁺) [mol %] in the different zones are summarized in the following table 4.

**table 4**

| **example no.:** | **1** | **2** | **3** | **4** | **5** | **6** | **7** | **8** | **9** | **10** |
|---|---|---|---|---|---|---|---|---|---|---|
| mean molar conc. of alkali ions in the outer zone (before storage) | 0.0 | 0.0 | 0.05 | 0.1 | 0.0 | 0.0 | 0.0 | 0.0 | 0.0 | 0.05 |
| mean molar conc. of alkali ions in the inner zone (before storage) | 0.8 | 0.7 | 1.15 | 1.4 | 0.8 | 1.1 | 0.5 | 0.8 | 0.7 | 0.95 |
| mean molar conc. of alkali ions in the outer zone (after storage) | 0.0 | 0.0 | 0.06 | 0.1 | 0.0 | 0.0 | 0.0 | 0.0 | 0.0 | 0.06 |
| mean molar conc. of alkali ions in the inner zone (after storage) | 0.8 | 0.7 | 1.14 | 1.4 | 0.8 | 1.1 | 0.5 | 0.8 | 0.7 | 0.94 |

The data surprisingly show that no detectable migration of alkali ions occurs after storage. They further show that for the particles according to the invention the mean concentration of alkali ions of the of outer zone relative to the mean concentration of alkali ions of the inner zone is 10 % or less. It was not expected in view of the state of the art that by this method of producing glass filler materials the particles outer zone could have a depth of up to 1.5 µm.

The composite materials made with the fillers according to the invention have good handling characteristics and the cured plastic polymers made from these composites exhibit very good mechanical properties as strength, hardness, elastic modulus and wear resistance. Additionally, they have an increased shelf life and polymerize entirely.

## Claims

1. Glass filler material for use in dental composites and dental restorations comprising
a) 65 - 99.95 mol% silicon dioxide (SiO₂),
b) 0-15 mol% aluminum and/or boron oxide (Al₂O₃, B₂O₃),
c) 0 - 30 mol % zirconium and/or titanium and/or hafnium oxide (ZrO₂, TiO₂, HfO₂),Y₂O₃ and/or Sc₂O₃ and/or La₂O₃ and/or CeO₂ and/or other lanthanide oxides
d) 0.05 - 4 mol% alkali metal oxides (Na₂O, Li₂O, K₂O, Rb₂O, Cs₂O)
e) 0-25 mol% earth alkali metal oxides (MgO, CaO, SrO, BaO)
wherein the glass filler particles have an average particle size of 0.1 - 20 µm and wherein these particles have an inner zone and an outer zone up to 1.5 µm and wherein the mean concentration of alkali ions of the outer zone relative to the mean concentration of alkali ions of the inner zone is 10 % or less and the alkali ions of the inner zone do not significantly migrate to the outer zone.

2. Glass filler material according to claim 1,
wherein the concentration of e) alkali metal oxides is not over 2 mol%.

3. Glass filler material according to claim 1 or 2,
wherein the glass filler particles have an average particle size of 0.5 to 3 µm.

4. Glass filler material according to one of the claims 1 to 3,
wherein the maximal particle size is up to 100 µm.

5. Glass filler material according to one of the claims 1 to 4,
wherein the refractive index n_{D} of the glass filler material n_{D} is in the range of 1.49 to 1.55.

6. A method for producing a glass filler material for use in dental composites and dental restorations with an average particle size of 0.1 to 20 µm by
a) melting a composition of 65 - 99.95 mol% SiO₂, 0-15 mol% Al₂O₃ and/or B₂O₃, 0-30 mol% ZrO₂ and/or TiO₂ and/or HfO₂, 0.05 - 4 mol% alkali metal oxides, 0 - 25 mol% earth alkali oxides, 0 - 30 mol% Y₂O₃ and/or Sc₂O₃ and/or La₂O₃ and/or Ce₂O₃ and/or other lanthanide oxides at a temperature of 1200 -1800 °C for at least 30 minutes,
b) crushing the melted glass by transferring into cold water or on metal rollers,
c) milling the glass granulate obtained by b) to a mean particle size of d₅₀ from 0.1 to 20 µm,
d) dealkalizing the glass powder in excess with a dealkalizing agent,
e) removing the dealkalizing agent and washing the glass powder with a polar solvent until the filtrate reacts neutral,
f) drying the glass powder at a temperature of 200 to 1100 °C for at least 30 minutes.

7. A method according to claim 6,
wherein the melting temperature is from 1400 to 1700 °C.

8. A method according to claim 6 or 7,
wherein the dealkalizing agent is an acidic composition.

9. A method according to one of the claims 6 to 8,
wherein the dealkalizing agent is an inorganically or organically acid.

10. A method according to one of the claims 6 to 9,
wherein the polar solvent consists of water or a mixture of water with other polar solvents.

11. A method according to one of the claims 6 to 10,
wherein is dealkalizing is performed at temperatures of 50 to 200 °c.

12. A method according to one of the claims 6 to 11,
wherein the ratio of the glass powder to the dealkalizing agent is 1:1 to 1:1000.

13. A polymerizable dental material containing:
a) 3 - 80 wt.% of one or more cationically curable monomers.
b) 3-90 wt.% of the glass filler material of claim 1 to 5,
c) 0 - 90 wt.% of one or more radio-opaque fillers,
d) 0.01- 25 wt.% of initiators, retarders and/or accelerators,
e) 0 - 25 wt.% of auxiliary agents

14. A polymerizable dental material according to claim 13,
wherein the curable monomer is an epoxide monomer.

15. Use of a glass filler material according to one of the claims 1 to 5,
for dental filling materials, dental cements, dental bonding materials, dental restorative materials.

## Patentansprüche

1. Glasfüllmaterial zur Verwendung in Dentalverbundstoffen und Dentalwiederherstellungen, umfassend
a) 65 - 99,95 Mol-% Siliciumdioxid (SiO₂),
b) 0 - 15 Mol-% Aluminium- und/oder Boroxid (Al₂O₃, B₂O₃) ,
c) 0 - 30 Mol-% Zirconium- und/oder Titan- und/oder Hafniumoxid (ZrO₂, TiO₂, HfO₂), Y₂O₃ und/oder Sc₂O₃ und/oder La₂O₃ und/oder CeO₂ und/oder andere Lanthanidoxide
d) 0,05 - 4 Mol-% Alkalimetalloxide (Na₂O, Li₂O, K₂O, Rb₂O, CS₂O)
e) 0 - 25 Mol-% Erdalkalimetalloxide (MgO, CaO, SrO, BaO)
wobei die Glasfüllmaterialteilchen eine mittlere Teilchengröße von 0,1 - 20 µm aufweisen und wobei diese Teilchen eine innere Zone und eine äußere Zone von bis zu 1,5 µm aufweisen und wobei die mittlere Konzentration von Alkaliionen der äußeren Zone mit Bezug auf die mittlere Konzentration von Alkaliionen der inneren Zone 10 % oder weniger beträgt und die Alkaliionen der inneren Zone nicht signifikant zur äußeren Zone migrieren.

2. Glasfüllmaterial nach Anspruch 1, wobei die Konzentration von e) Alkalimetalloxiden nicht mehr als 2 Mol-% beträgt.

3. Glasfüllmaterial nach Anspruch 1 oder 2, wobei die Glasfüllmaterialteilchen eine mittlere Teilchengröße von 0,5 bis 3 µm aufweisen.

4. Glasfüllmaterial nach einem der Ansprüche 1 bis 3, wobei die maximale Teilchengröße bis zu 100 µm beträgt.

5. Glasfüllmaterial nach einem der Ansprüche 1 bis 4, wobei der Brechungsindex n_{D} des Glasfüllmaterials n_{D} im Bereich von 1,49 bis 1,55 liegt.

6. Verfahren für die Herstellung eines Glasfüllmaterials zur Verwendung in Dentalverbundstoffen und Dentalwiederherstellungsmaterialien mit einer mittleren Teilchengröße von 0,1 bis 20 µm durch
a) Schmelzen einer Zusammensetzung von 65 - 99,95 Mol-% SiO₂, 0 - 15 Mol-% Al₂O₃ und/oder B₂O₃, 0 - 30 Mol-% ZrO₂ und/oder TiO₂ und/oder HfO₂, 0,05 - 4 Mol-% Alkalimetalloxiden, 0 - 25 Mol-% Erdalkalimetalloxiden, 0 - 30 Mol-% Y₂O₃ und/oder Sc₂O₃ und/oder La₂O₃ und/oder Ce₂O₃ und/oder anderen Lanthanidoxiden bei einer Temperatur von 1200 - 1800 °C für mindestens 30 Minuten
b) Zerkleinern des geschmolzenen Glases durch Übertragen in kaltes Wasser oder auf Metallwalzen,
c) Mahlen des durch b) erhaltenen Glasgranulats auf eine mittlere Teilchengröße von d₅₀ von 0,1 bis 20 µm
d) Entalkalisieren des Glaspulvers im Überschuss mit einem Entalkalisiermittel,
e) Entfernen des Entalkalisiermittels und Waschen des Glaspulvers mit einem polaren Lösungsmittel, bis das Filtrat neutral reagiert,
f) Trocknen des Glaspulvers bei einer Temperatur von 200 bis 1100 °C für mindestens 30 Minuten.

7. Verfahren nach Anspruch 6, wobei die Schmelztemperatur 1400 bis 1700 °C beträgt.

8. Verfahren nach Anspruch 6 oder 7, wobei das Entalkalisiermittel eine saure Zusammensetzung ist.

9. Verfahren nach einem der Ansprüche 6 bis 8, wobei das Entalkalisiermittel eine anorganische oder organische Säure ist.

10. Verfahren nach einem der Ansprüche 6 bis 9, wobei das polare Lösungsmittel aus Wasser oder einer Mischung von Wasser mit anderen polaren Lösungsmitteln besteht.

11. Verfahren nach einem der Ansprüche 6 bis 10, wobei die Entalkalisierung bei einer Temperatur von 50 bis 200 °C durchgeführt wird.

12. Verfahren nach einem der Ansprüche 6 bis 11, wobei das Verhältnis des Glaspulvers zum Entalkalisiermittel 1:1 bis 1:1000 ist.

13. Polymerisierbares Dentalmaterial enthaltend:
a) 3 - 80 Gew.-% eines oder mehrerer kationisch aushärtbarer Monomere,
b) 3 - 90 Gew.-% des Glasfüllmaterials nach Anspruch 1 bis 5,
c) 0 - 90 Gew.-% eines oder mehrerer radioopaker Füllstoffe
d) 0,01 - 25 Gew.-% Initiatoren, Verzögerungsmittel und/oder Beschleunigungsmittel,
e) 0 - 25 Gew.-% Hilfsmittel.

14. Polymerisierbares Dentalmaterial nach Anspruch 13, wobei das aushärtbare Monomer ein Epoxidmonomer ist.

15. Verwendung eines Glasfüllmaterials nach einem der Ansprüche 1 bis 5, für Dentalfüllmaterialien, Dentalzemente, Dentalbindungsmaterialien, Dentalwiederherstellungsmaterialien.

## Revendications

1. Matériau de remplissage à base de verre pour une utilisation dans des composites dentaires et des restaurations dentaires comprenant
a) 65-99,95 % en moles de dioxyde de silicium (SiO₂),
b) 0-15 % en moles d'oxyde d'aluminium et/ou de bore (Al₂O₃, B₂O₃) ,
c) 0-30 % en moles d'oxyde de zirconium et/ou de titane et/ou d'hafnium (ZrO₂, TiO₂, HfO₂), Y₂O₃ et/ou Sc₂O₃ et/ou La₂O₃ et/ou CeO₂ et/ou d'autres oxydes de lanthanides,
d) 0,05-4 % en moles d'oxydes de métaux alcalins (Na₂O, Li₂O, K₂O, Rb₂O, C_{S2}O),
e) 0-25 % en moles d'oxydes de métaux alcalino-terreux (MgO, CaO, SrO, BaO)
dans lequel les particules de remplissage à base de verre possèdent une taille moyenne de particule de 0,1 à 20 µm et dans lequel ces particules comportent une zone interne et une zone externe allant jusqu'à 1,5 *µ*m et dans lequel la concentration moyenne d'ions alcalins de la zone externe rapportée à la concentration moyenne d'ions alcalins de la zone interne est de 10 % ou moins et les ions alcalins de la zone interne ne migrent pas significativement vers la zone externe.

2. Matériau de remplissage à base de verre selon la revendication 1,
dans lequel la concentration des e) oxydes de métaux alcalins ne dépasse pas 2 % en moles.

3. Matériau de remplissage à base de verre selon la revendication 1 ou 2,
dans lequel les particules de remplissage à base de verre possèdent une taille moyenne de particule de 0,5 à 3 µm.

4. Matériau de remplissage à base de verre selon l'une des revendications 1 à 3,
dans lequel la taille maximale de particule atteint 100 µm.

5. Matériau de remplissage à base de verre selon l'une des revendications 1 à 4,
dans lequel l'indice de réfraction n_{D} du matériau de remplissage à base de verre se situe dans la gamme de 1,49 à 1,55.

6. Procédé de fabrication d'un matériau de remplissage à base de verre pour une utilisation dans des composites dentaires et des restaurations dentaires avec une taille moyenne de particule de 0,1 à 20 µm par
a) fusion d'une composition de 65-99,95 % en moles de SiO₂, 0-15 % en moles de Al₂O₃ et/ou B₂O₃, 0-30 % en moles de ZrO₂ et/ou TiO₂ et/ou HfO₂, 0,05-4 % en moles d'oxydes de métaux alcalins, 0-25 % en moles d'oxydes de métaux alcalino-terreux, 0-30 % en moles de Y₂O₃ et/ou Sc₂O₃ et/ou La₂O₃ et/ou Ce₂O₃ et/ou d'autres oxydes de lanthanides à une température de 1200-1800°C pendant au moins 30 minutes,
b) concassage du verre fondu par transfert dans de l'eau froide ou sur des rouleaux métalliques,
c) broyage du granulé de verre obtenu en b) jusqu'à une taille moyenne de particule d₅₀ de 0,1 à 20 µm,
d) désalcalinisation de la poudre de verre en excès avec un agent de désalcalinisation,
e) retrait de l'agent de désalcalinisation et lavage de la poudre de verre avec un solvant polaire jusqu'à ce que le filtrat réagisse de façon neutre,
f) séchage de la poudre de verre à une température de 200 à 1100°C pendant au moins 30 minutes.

7. Procédé selon la revendication 6,
dans lequel la température de fusion va de 1400 à 1700°C.

8. Procédé selon la revendication 6 ou 7,
dans lequel l'agent de désalcalinisation est une composition acide.

9. Procédé selon l'une des revendications 6 à 8,
dans lequel l'agent de désalcalinisation est un acide inorganique ou organique.

10. Procédé selon l'une des revendications 6 à 9,
dans lequel le solvant polaire consiste en de l'eau ou un mélange d'eau avec d'autres solvants polaires.

11. Procédé selon l'une des revendications 6 à 10,
dans lequel la désalcalinisation est effectuée à des températures de 50 à 200°C.

12. Procédé selon l'une des revendications 6 à 11,
dans lequel le rapport de la poudre de verre à l'agent de désalcalinisation va de 1:1 à 1:1000.

13. Matériau dentaire polymérisable contenant :
a) 3-80 % en poids d'un ou plusieurs monomères à durcissement cationique,
b) 3-90 % en poids du matériau de remplissage à base de verre des revendications 1 à 5,
c) 0-90 % en poids d'une ou plusieurs charges radio-opaques,
d) 0,01-25 % en poids d'initiateurs, de retardateurs et/ou d'accélérateurs,
e) 0-25 % en poids d'agents auxiliaires.

14. Matériau dentaire polymérisable selon la revendication 13,
dans lequel le monomère durcissable est un monomère époxyde.

15. Utilisation d'un matériau de remplissage à base de verre selon l'une des revendications 1 à 5, pour des matériaux de remplissage dentaire, des ciments dentaires, des matériaux de colle dentaire, des matériaux de restauration dentaire.
